# EUROPÄISCHE PATENTSCHRIFT

(11) **EP 1 154 732 B1**
(45) Veröffentlichungstag und Bekanntmachung des Hinweises auf die Patenterteilung: **26.05.2004**
(21) Anmeldenummer: 00900442.5
(22) Anmeldetag: 03.01.2000
(51) Int. Cl.: A61F 2/00

(54) **IMPLANTIERBARER BETÄTIGUNGSMECHANISMUS**
IMPLANTABLE ACTUATING MECHANISM
MECANISME DE COMMANDE IMPLANTABLE

(30) Priorität: 22.02.1999 AT 29099
(43) Veröffentlichungstag der Anmeldung: 21.11.2001
(73) Patentinhaber: Pregenzer, Bruno, 6414 Mieming (AT); Stenzl, Arnulf, 72076 Tübigen (DE)
(72) Erfinder: Pregenzer, Bruno, 6414 Mieming (AT); Stenzl, Arnulf, 72076 Tübigen (DE)
(74) Vertreter: Hofinger, Engelbert, Dr.Dr.
(86) Internationale Anmeldenummer: PCT/AT2000/000001
(87) Internationale Veröffentlichungsnummer: WO 2000/049969

(56) Entgegenhaltungen:
- WO-A-96/01597
- DE-A- 2 806 405
- US-A- 4 453 536
- US-A- 5 518 504

## Beschreibung

Die Erfindung betrifft einen implantierbaren, zwischen zwei stabilen Stellungen schaltbaren Betätigungsmechanismus für ein zwischen zwei Zuständen oder Stellungen schaltbares Implantat, mit einem Stützelement, das eine Montageplatte zur Abstützung an einem Knochen aufweist und mit einem relativ zum Stützelement bewegbaren, federbeaufschlagten Betätigungselement, das sich durch das Stützelement erstreckt und mittels eines Seiles mit einem einen Wechsel zwischen den beiden Stellungen des Implantates auslösenden, jenseits des Stützelementes vorgesehenen Aktivierungsteil verbunden ist.

Zur Verhinderung der Harninkontinenz ist aus der EP-A 639 355 eine die Harnröhre untergreifende Bandschlaufe aus einem körperverträglichen Fremdmaterial vorgesehen, wobei die Bandenden an einer höheren Stelle im Körper fixiert werden, und der Mittelbereich der Bandschleife eine mit einem Fluid füllbare Kammer darstellt, wobei die Menge des eingefüllten Fluids die Auflagehöhe der Harnröhre bestimmt und daher ebenfalls justiert werden kann. In der EP-A 639 355 ist auch angeführt, daß schon Faszien für die Bildung einer Bandschlaufe verwendet worden sind, die im Körper fixiert. werden. Eine spätere Korrektur aufgrund von Veränderungen ist allerdings kaum möglich.

Einen weiteren implantierbaren Betätigungsmechanismus zeigt die US 5,518,504. Hier wird eine die Harnröhre untergreifende Bandschlaufe ebenfalls aus einer hochgezogenen Ruhestellung mittels eines hydraulischen Systems abgesenkt, das ein Kolbenpumpenpaar umfaßt.

Die Nachteile dieser Einrichtungen liegen in der Verwendung eines Druckfluids und der zu verlegenden Schläuche, der Gefahr des Leckwerdens, der Notwendigkeit eines zu implantierenden Vorratsbehälters usw.

Ein Betätigungsmechanismus der eingangs genannten Art für eine Absperreinrichtung für natürliche, röhrenartige Körperorgane ist beispielsweise der US-A 4,453,536 zu entnehmen. Das Stützelement wird je nach Verwendungszweck an einer geeigneten Stelle in den Körper eingesetzt, sodaß das Betätigungselement durch die Haut erreichbar angeordnet ist und durch Druck von außen im Stützelement bewegt werden kann. Insbesondere wird das Stützelement in der Bauchdecke im Becken- oder Schambereich implantiert, wobei eine Montageplatte an der Innenseite und ein im Abstand einstellbarer Flansch an der Außenseite der Bauchmuskulatur anliegen. Das röhrenförmige Körperorgan wird von einem dreifingrigen Klemmelement umgriffen, wobei der mittlere Finger über einen Seilzug mit dem unter der Haut angeordneten Betätigungselement verbunden und durch dessen Rückstellfeder in einer Sperrstellung gehalten wird, in der das röhrenförmige Körperorgan flach gedrückt ist. Beim Drücken des Betätigungselementes führt der aus dem Stützelement nach innen vorstehende Aktivierungsteil eine lineare Bewegung aus, die durch den Seilzug an den mittleren Finger übertragen wird, und das Klemmelement öffnet, wobei die Offenstellung durch einen seitlichen Druck auf das Betätigungselement vorübergehend fixiert werden kann. Eine reelle Einbausituation ist nicht gezeigt.

Die Erfindung hat es sich nun zur Aufgabe gestellt, einen implantierbaren Betätigungsmechanismus der eingangs genannten Art zu schaffen, der ohne hydraulisches System ein zwischen zwei Zuständen oder Stellungen schaltbares Implantat betätigt, und insbesondere eine Harninkontinenz verhindert. Unterschiedlichen Gegebenheiten kann erfindungsgemäß dadurch weitestgehend Rechnung getragen werden, daß das Stützelement einen gebogenen Endbereich aufweist, in dem das Seil zum Aktivierungsteil geführt ist.

Eine bevorzugte Ausführung des Betätigungsmechanismus sieht vor, daß der Aktivierungsteil eine Aufnahme für die beiden Enden einer eine Körperröhre untergreifende Faszie aufweist. Eine derartige Aufnahme umfaßt insbesondere eine Kombination aus Formschluß und Kraftschluß, um eine mehrjährige störungsfreie Verbindung sicherzustellen. Der Aktivierungsteil weist zwei oder drei Anlageplatten od.dgl. auf, zwischen denen die Faszienenden gehalten werden, wobei für den Formschluß Erhebungen und korrespondierende Vertiefungen vorgesehen sind. Die Erhebungen können einerseits pyramidenförmig ausgebildet sein und in die Faszie einstechende Spitzen aufweisen, und andererseits zylindrische Stifte bilden, die in Bohrungen oder Durchbrechungen ragen. Für den zusätzlichen Kraftschluß kann weiters ein hinterschnittener, federnder Rastzapfen vorgesehen sein, der in einer der Durchbrechungen unter Klemmung der Faszienenden verrastet.

Eine Justierung der über das Stützelement vorstehenden Länge des Aktivierungselementes kann mittels einer Stellschraube erfolgen, auf die an der Druckfläche des Betätigungselementes zugegriffen werden kann, und die durch die Haut zugänglich ist.

Zur Abschirmung des Seilzuges ist bevorzugt zwischen dem Stützelement und dem Aktivierungsteil ein Faltenbalg vorgesehen.

Für eine aktive, d.h. von außen beeinflußbare Schließung und Öffnung der Harnröhre durch eine Hebung und Senkung einer die Harnröhre untergreifenden Faszie, die entweder eine Änderung des vesico-urethralen Winkels oder durch Hochheben der Urethra deren Abklemmen bewirkt, ist vorgesehen, daß die beiden Enden der Faszie mit einem am Schambein fixierbaren Betätigungsmechanismus verbunden sind, wobei sowohl die obere Schließstellung als auch die untere Öffnungsstellung der Faszie jeweils durch eine der beiden stabilen Stellungen des Betätigungselementes definiert wird.

Nachstehend wird anhand der Fig. 1 bis 6 der beiliegenden Zeichnungen eine Ausführung des Betätigungsmechanismus näher beschrieben. Darin zeigen:
- Fig. 1: einen Längsschnitt durch einen Betätigungsmechanismus in einer ersten Stellung,
- Fig. 2: einen Längsschnitt durch den Betätigungsmechanismus in einer zweiten Stellung,
- Fig. 3: eine Einbaustellung,
- Fig. 4: eine vergrößerte Darstellung einer herzförmigen Führungsbahn,
- Fig. 5: eine Seitenansicht auf eine Faszienaufnahme, und
- Fig. 6: einen Schnitt durch eine zweite Ausführung einer Faszienaufnahme.

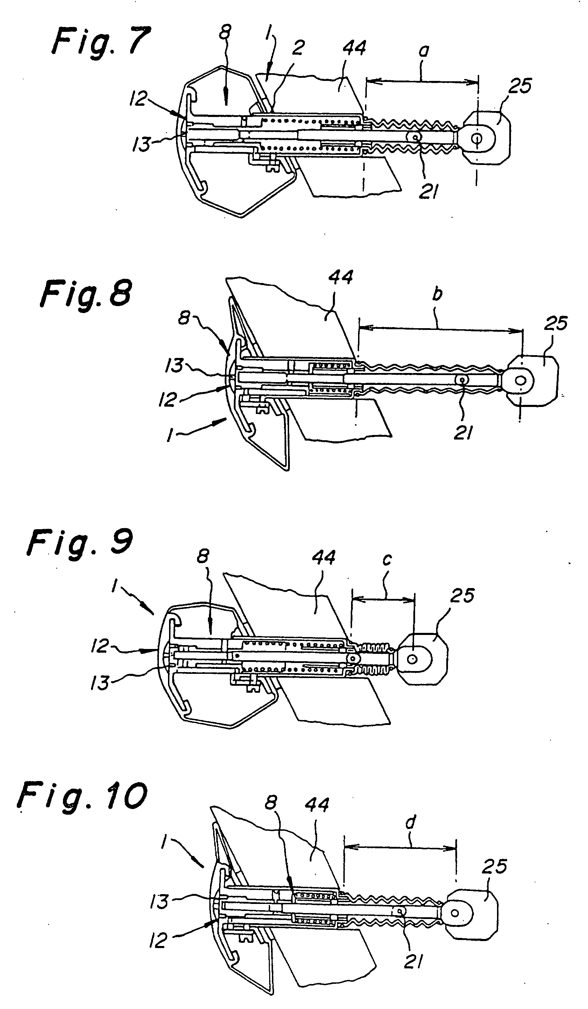

Des weiteren wird zum besseren Verständnis anhand einer in den Fig. 7 bis 10 der beiliegenden Zeichnungen dargestellten Ausführung, deren konstruktiver Aufbau nicht durch den Patentanspruch 1 umfasst ist, das Funktionsprinzip des Betätigungsmechanismus erläutert. Hierfür zeigen:
Fig. 7 und 8 Längsschnitte durch die beiden stabilen Stellungen des Betätigungsmechanismus, und
Fig. 9 und 10 Längsschnitte gemäß Fig. 7 und 8 mit verkürztem Aktivierungsteil.

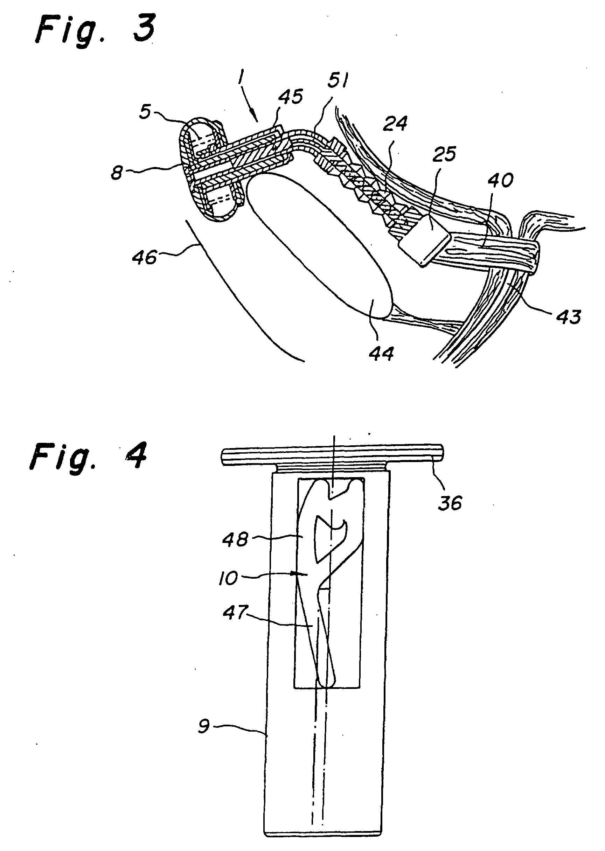

Ein erfindungsgemäßer Betätigungsmechanismus 1 dient zur Schaltung eines zwei stabile Schaltstellungen aufweisenden Implantates, insbesondere eines künstlichen Verschlusses eines Körperdurchganges, einer Körperöffnung usw., wie zum Beispiel der Harnröhre 43 (Fig. 3). Der Betätigungsmechanismus 1 ist ebenfalls an geeigneter Stelle implantierbar, und wird im Falle eines künstlichen Harnröhrenverschlusses am Schambein 44 bzw. der Symphyse abgestützt.

Der Betätigungsmechanismus 1 weist ein Stützelement 2 auf, das mit einer Montageplatte 3 und einer von einem Hüllrohr 45 umgebenden Führungshülse 4 versehen ist. Das Stützelement 2 weist einen gebogenen Endabschnitt 50 auf, wodurch der Betätigungsmechanismus 1, wie Fig. 3 zeigt, oberhalb des Schambeines angeordnet werden kann. Die Montageplatte 3 ist mit seitlichen senkrecht zur Zeichenebene abstehenden Befestigungslaschen versehen, die mit Knochenschrauben od.dgl. am Schambein festlegbar sind.

Der Montageplatte 3 ist weiters eine Klemmplatte 6 zugeordnet, wobei zwischen der Montageplatte 3 und der Klemmplatte 6 ein Faltenbalg bzw. Ballon 23 aus einem physiologisch verträglichen Kunststoff od.dgl. dichtend eingeklemmt ist, dessen anderer Rand an einem Flansch 36 eines Betätigungselementes 8 dichtend gehalten ist, das in der Führungshülse 4 des Stützelementes 2 verschiebbar geführt ist.

Das Betätigungselement 8 weist eine in der Führungshülse 4 verschiebbare innere Hülse 9 auf, die gegen Verdrehen gesichert ist, und in der eine Stellschraube 13 drehbar gehalten ist. Die Stellschraube 13 ist mit einer Ringnut 14 versehen, in die ein in der Hülse 9 gelagerter Führungsstift 15 eingreift. Der Kopf der Stellschraube 13 bildet eine Druckfläche des Betätigungselementes und überdeckt den Rand des Faltenbalges 23. Die Stellschraube 13 weist einen Schlitz zum Angriff eines Werkzeuges auf. Die Stellschraube 13 ist hohl und trägt im unteren Bereich einen Innengewindeabschnitt 16, der auf einen Gewindestab 18 aufgeschraubt ist. Die Verbindung zwischen dem aus der Stellschraube 13 vorstehenden Teil des Gewindestabs 18 und einer Aufnahme 25, deren Funktion später beschrieben wird, wird durch einen Seilzug gebildet. Wird die Stellschraube 13 mit Hilfe eines in den Schlitz eingreifenden Werkzeugs verdreht, so bewirken die axiale Festlegung der Stellschraube 13 durch den Stift 15 eine axiale Verstellung des Gewindestabes 18 samt Seilzug.

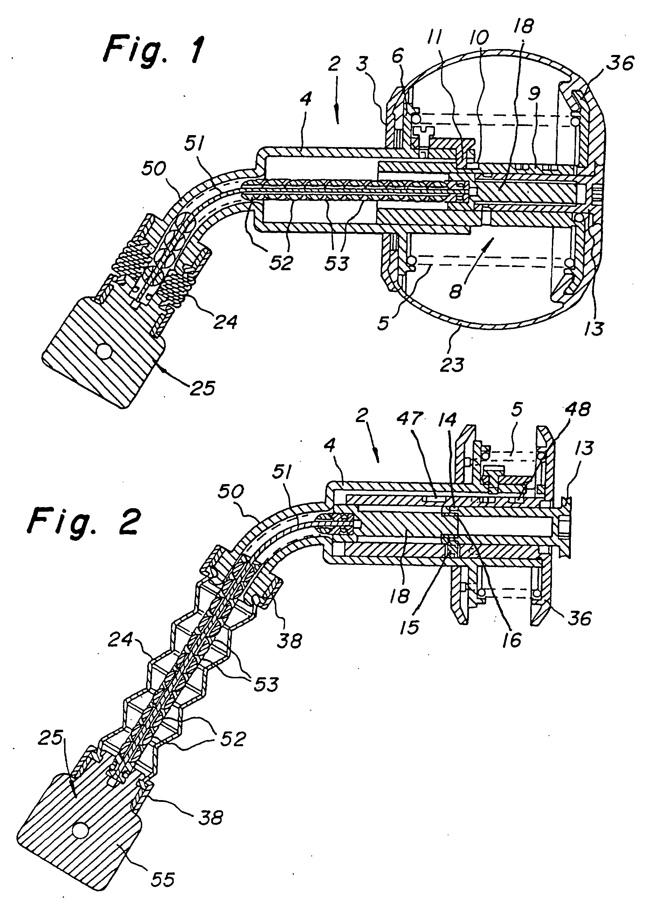

Zwischen der inneren Hülse 9 und der Führungshülse 4 ist eine Rückstellfeder 5 angeordnet, die das Betätigungselement 8 in die in Fig. 1 gezeigte Stellung beaufschlagt, in der das Betätigungselement 8 zum Stützelement 2 hin bewegbar ist, und die Aufnahme 25 den geringsten Abstand zur Führungshülse 4 aufweist. Der Faltenbalg 23 ist auseinandergezogen, und ein zweiter Faltenbalg 24 zwischen zwei Überwurfmuttern 38 der Aufnahme 25 und der Führungshülse 4 stark gefaltet.

Bevorzugt ist innerhalb des Faltenbalges 24 bzw. in seiner Wandung eine Rückstellfeder vorgesehen, die der innerhalb des ersten Faltenbalges 23 angeordneten Rückstellfeder 5 entgegenwirkt, aber schwächer als diese ist.

Die in der Führungshülse 4 verschiebbare innere Hülse 9 weist eine herzförmige Führungsbahn 10 auf, die aus Fig. 4 ersichtlich ist, in der ein querbeweglich angelenkter Führungsstift 11 entlang gleitet, und zwei stabile Stellungen einnehmen kann. In der einen stabilen Stellung liegt der Führungsstift 11 am unteren Ende des unteren Bereiches 47 (Fig. 1) und in der anderen Stellung in der mittleren Vertiefung der Herzform im oberen Bereich 48. Da die Längsschnitte der Fig. 1 und 2 in einer Axialebene liegen, sind nur kurze Bereiche der Führungsbahn 10 ersichtlich, wobei Fig. 2 eine maximal komprimierte Stellung des Betätigungselementes 8 zeigt, in der der Führungsstift 11 im oberen Bereich 48 seitlich ausgeschwenkt ist. (In Fig. 2 ist der in Fig. 1 etwa ballonartig ausgebildete erste Faltenbalg 23 der Einfachkeit halber nicht dargestellt.)

Der Führungsstift 11 ragt durch einen gebogenen Schlitz der Führungshülse 4 in die Führungsbahn 10. Die angenäherte Herzform bewirkt am oberen Ende eine Umlenkung des Führungsstiftes 11 in ihren kurzen Mittelabschnitt, sodaß bei Aufhebung des Drucks auf das Betätigungselement 8 die innere Hülse 9 aufgrund der Wirkung der Rückstellfeder 5 nur eine geringe Rückbewegung ausführt, und in der zweiten stabilen Stellung verbleibt, in der der Faltenbalg 23 stark gefaltet, der Faltenbalg 24 weit auseinandergezogen ist, und die am Ende des Seilzugs angeordnete Aufnahme vom Ende der Führungshülse 4 einen großen Abstand aufweist. Wird das Befestigungselement nochmals etwas gedrückt, so gleitet der Führungsstift 11 im oberen Bereich 48 der angenäherten Herzform weiter, bis der zweite obere Umlenkbereich erreicht ist, und von dort in die in Fig. 1 gezeigte tiefste Ausgangsstellung, wenn das Betätigungselement 8 unter der Wirkung der Feder 5 in die erste stabile Stellung zurückgeführt wird, in der sich die Aufnahme 25 wieder an die Führungshülse 4 angenähert hat. Der Boden der Führungsbahn 10 kann zumindest im oberen Bereich 48 in jedem Wendepunkt abgestuft sein, um zu vermeiden, daß der in diesem Fall federbeaufschlagte Führungsstift 11 nach der falschen Seite gleitet.

Das Seil 51 kann direkt im gebogenen Endabschnitt 50 geführt sein, oder aber, wie in den Fig. 1 und 2 dargestellt, in einer Seilhülle angeordnet sein, wenn beispielsweise zwischen den für das Seil 51 und den gebogenen Endabschnitt 50 verwendeten Materialien eine zu hohe Reibung gegeben wäre. Da die Seilhülle möglichst flexibel sein muß, um mit dem Seil 51 durch den gebogenen Endabschnitt 50 zu gleiten, ist die Seilhülle 51 bevorzugt aus aneinandergereihten Kugeln 52 und Zwischenscheiben 53 zusammengesetzt, die jeweils zwei konkave Anlageflächen für die Kugeln 52 aufweisen. Die Zwischenscheiben 53 sind dadurch auf den Kugeln 52 begrenzt verschwenkbar angeordnet, und der Seilzug kann durch den gebogenen Endabschnitt bewegt werden, wie der Vergleich der Fig. 1 und 2 zeigt.

Die Fig. 7 bis 10 zeigen schematisch einen Betätigungsmechanismus 1, der, wie erwähnt, nicht durch Patentanspruch 1 umfaßt ist, in vier unterschiedlichen Positionen. In Fig. 7 ist der Betätigungsmechanismus in seiner ersten stabilen Stellung und der Gewindestab 18 am äußeren Ende der Stellschraube 13. Die Aufnahme 25 liegt daher in einem kleinen Abstand a von der Führungshülse 4 des Stützelements 2. Wird das Betätigungselement 8 in die in Fig. 8 gezeigte zweite stabile Stellung überführt, so vergrößert sich der Abstand der Aufnahme 25 von der Führungshülse 4 auf das Maß b.

In Fig. 9, die der Fig. 1 entspricht, ist der Gewindestab 18 weitest möglich in die Stellschraube 13 eingezogen, sodaß der Abstand c der Aufnahme von der Führungshülse 4 um die gesamte Verstelllänge des Betätigungselementes 8 kleiner ist als der Abstand e in Fig. 7.

Fig. 10 zeigt wiederum die zweite stabile Stellung des Betätigungselementes 8 bei eingezogenem Gewindestab 18. Der größtmögliche Abstand d liegt zwischen den Abständen a und c der Fig. 7 und 8.

Fig. 3 zeigt eine bevorzugte Anwendung des erfindungsgemäßen Betätigungsmechanismus 1 zur Senkung eines operativ angehobenen Harnröhrenansatzes, d.h. zum künstlichen Öffnen und Verschließen einer Harnröhre 43 nahe der Harnblase. Als künstliches Verschlußelement dient eine Faszie 40, die unterhalb der Harnröhre 43 angeordnet wird, und deren beiden Enden in der Aufnahme 25 des Betätigungselementes 8 fixiert werden.

In der ersten stabilen Stellung der implantierten, die Harnröhre 43 untergreifende Faszie 40 ist die Harnröhre angehoben und so geknickt, daß ein Harnabfluß nicht möglich ist. Das Betätigungselement 8 ist in seiner ersten stabilen Stellung gemäß Fig. 7, in der der Abstand a zwischen der Führungshülse 4 und der Aufnahme 25 gegeben ist. Wenn das Betätigungselement 8 durch äußere Druckeinwirkung auf die unter der Haut 46 liegende Druckfläche 12 in die in Fig. 8 gezeigte Stellung überführt wird, erhöht sich der Abstand der Aufnahme 25 auf das Maß b und die Faszie 40 wird um diese Differenz freigegeben, sodaß der Harnröhrenansatz abgesenkt und die Harnröhre 43 geöffnet wird. Dies stellt die zweite stabile Stellung des Implantates dar (Fig. 3). Eine nochmalige äußere Druckeinwirkung auf die Druckfläche 12 löst die Rückstellung in die Position nach Fig. 7 aus.

Bei einer Verschlechterung der Gegebenheiten kann der Betätigungsmechanismus 1 nachgestellt werden, indem durch einen kleinen operativen Eingriff in Lokalanästhesie die Stellschraube 13 verdreht wird, bis die Faszie 40 um das benötigte Maß nachgezogen worden ist. Die Justierung kann maximal bis zur Position nach Fig. 9 erfolgen, und das Nachstellausmaß beträgt die Differenz der Abstände a und c, die realistischerweise etwa bis zu 10 mm betragen kann. Auch in dieser justierten Lage ist das Öffnen und Schließen der Harnröhre 43 in der beschriebenen Weise durchführbar.

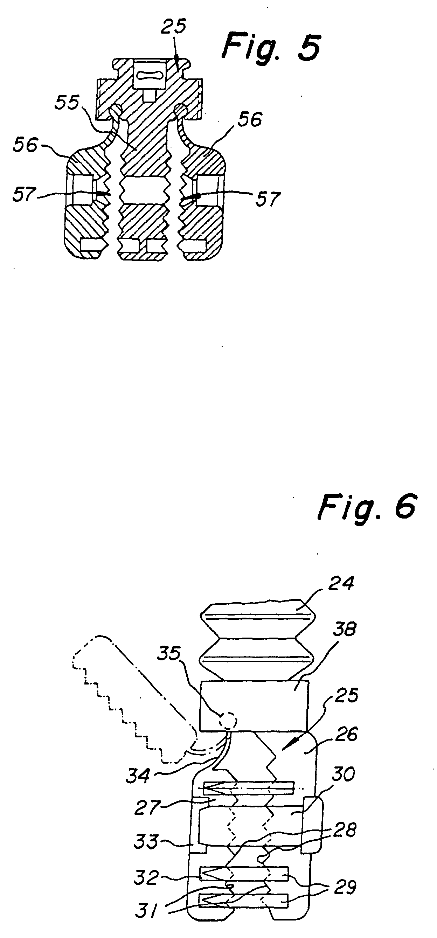

Fig. 5 zeigt eine Ausführung der Aufnahme 25 in Seitenansicht. Über das Gelenk 21 ist eine erste Anlageplatte 26 mit dem Betätigungselement 8 verbunden, an deren Kopfende eine zweite Anlageplatte 27 vorgesehen ist. Die zweite Anlageplatte 27 weist einen Zapfen 35 auf, der in eine Nut im Kopfende der Anlageplatte 26 eingesetzt ist, und mit dem sie über ein flexibles Band 34 verbunden ist. Beide Anlageplatten 26, 27 sind an den zueinander weisenden Flächen mit pyramidenförmigen Erhebungen 28 und Vertiefungen 31 versehen, zwischen denen die Enden der Faszie 40 angeordnet werden. Weiters stehen von der ersten Anlageplatte 26 Stifte 29 ab, die durch die Faszie durchgeführt werden, und deren gespitzte Enden in Bohrungen 32 der zweiten Anlageplatte 27 ragen. Als Verbindung zwischen den beiden Anlageplatten 26, 27 ist ein hinterschnittener Rastzapfen 30 vorgesehen, der in einer Durchbrechung 33 der zweiten Anlageplatte 27 verrastet. Anstelle des Rastzapfens 30 könnte auch eine Niet od.dgl. vorgesehen sein. Am Kopfende der ersten Anlageplatte 26 ist ein Gewinde ausgebildet, auf das die den Faltenbalg 24 an der Aufnahme 25 einspannende Überwurfmutter 38 aufgeschraubt ist.

Wie Fig. 6 zeigt, kann die Aufnahme 25 auch dreiteilig ausgebildet sein, und eine mittlere Anlageplatte 55 (Fig. 1, 2) aufweisen, die mit dem Betätigungselement 8 gekoppelt ist. An der mittleren Anlageplatte 55 sind beidseitig äußere Anlageplatten 56 angelenkt, so daß jedes Ende der Faszie 40 in einen separaten Aufnahmeschlitz 57 eingesetzt werden kann. Auch an diesen Anlageplatten sind bevorzugt ineinandergreifende Erhebungen und Vertiefungen vorgesehen.

## Patentansprüche

1. Implantierbarer, zwischen zwei stabilen Stellungen schaltbarer Betätigungsmechanismus (1) für ein zwischen zwei Zuständen oder Stellungen schaltbares Implantat, mit einem Stützelement (2), das eine Montageplatte (3) zur Abstützung an einem Knochen aufweist und mit einem relativ zum Stützelement (2) bewegbaren, federbeaufschlagten Betätigungselement (8), das sich durch das Stützelement (2) erstreckt und mittels eines Seiles (51) mit einem einen Wechsel zwischen den beiden Stellungen des Implantates auslösenden, jenseits des Stützelementes (2) vorgesehenen Aktivierungsteil verbunden ist, **dadurch gekennzeichnet, daß** das Stützelement (2) einen gebogenen Endbereich (50) aufweist, in dem das Seil (51) zum Aktivierungsteil geführt ist.

2. Betätigungsmechanismus nach Anspruch 1, **dadurch gekennzeichnet, daß** der Aktivierungsteil eine Aufnahme (25) für die beiden Enden einer eine Körperröhre untergreifenden Faszie (40) aufweist.

3. Betätigungsmechanismus nach Anspruch 2, **dadurch gekennzeichnet, daß** der Aufnahme (25) eine erste Anlageplatte (26) und eine gegenüberliegende zweite Anlageplatte (27) aufweist, die mit korrespondierenden Erhebungen und Vertiefungen versehen sind.

4. Betätigungsmechanismus nach Anspruch 2, **dadurch gekennzeichnet, daß** die Aufnahme (25) eine erste, mittlere Anlageplatte (55) und zwei äußere Anlageplatten aufweist, die mit korrespondierenden Erhebungen und Vertiefungen versehen sind.

5. Betätigungsmechanismus nach Anspruch 3 oder 4, **dadurch gekennzeichnet, daß** ein Teil der Erhebungen in Spitzen (28) endet.

6. Betätigungsmechanismus nach einem der Ansprüche 3 bis 5, **dadurch gekennzeichnet, daß** ein zweiter Teil der Erhebungen durch zylindrische Stifte (29) gebildet ist, die in Bohrungen (32) eingreifen.

7. Betätigungsmechanismus nach Anspruch 1, **dadurch gekennzeichnet, daß** das Seil (51) in einer Seilhülle angeordnet ist, die aneinandergereihte Kugeln (52) und Zwischenscheiben (53) mit kugeligen Anlageflächen aufweist.

8. Betätigungsmechanismus nach einem der Ansprüche 1 bis 7, **dadurch gekennzeichnet, daß** zwischen dem Stützelement (2) und dem Aktivierungsteil ein Faltenbalg (24) vorgesehen ist.

9. Implantierbare Einrichtung zur Verhinderung der Harninkontinenz, mit einer die Harnröhre (43) untergreifenden Faszie (40), die zur Hebung des Harnröhrenansatzes aus einer unteren in eine obere Stellung überführbar ist, **dadurch gekennzeichnet, daß** die beiden Enden der Faszie (40) mit einem am Schambein (44) fixierbaren Betätigungsmechanismus (1) nach einem der Ansprüche 1 bis 8 verbunden sind, wobei beide Stellungen der Faszie (40) jeweils durch eine stabile Stellung des Betätigungselementes (8) definiert sind.

## Claims

1. Implantable actuating mechanism (1) switchable between two stable settings for an implant to be switched between two stable settings, comprising a support element (2) having a mounting plate (3) for supporting on a bone of a patient, and a spring-activated actuating element (8) movably disposed relative to the support element (2) and guided by the support element (2), wherein the actuating element (8) is connected via a cable (51) to an activation part situated on an opposite side of the support element (2) and triggering the switch between the two settings of the implant, and wherein the support element (2) has a curved end portion (50) through which the cable (51) is guided to the activation part.

2. The actuating mechanism according to claim 1, wherein the activation part has a holder (25) for holding two ends of a fascia (40) engaging under a tubular body organ.

3. The actuating mechanism according to claim 2, wherein the holder (25) includes a first bearing plate (26) and a second, opposite bearing plate (27), and the bearing plates are formed with corresponding elevations and depressions.

4. The actuating mechanism according to claim 3, wherein the holder (25) has a first, central bearing plate (55) and two outer bearing plates formed with corresponding elevations and depressions.

5. The actuating mechanism according to claim 3 or 4, wherein one part of the elevations end in points (28).

6. The actuating mechanism according to one of claims 3 to 5, wherein another part of elevations are formed by cylindrical pins (29) engaging with bores (32).

7. The actuating mechanism according to claim 1, wherein the cable (51) is disposed in a cable sheath having a succession of balls (52) and intermediate disks with spherical bearing surfaces.

8. The actuating mechanism according to one of claims 1 to 7, wherein a bellows (24) is disposed between said support element and said activation part.

9. An implantable device for preventing urinary incontinence comprising a fascia (40) configured to engage under a patient's urethra (43) and to be moved from a lower position to an upper position for lifting the proximal urethra, and an actuating mechanism (1) according to one of claims 1 to 8 configured to be fixed to the patient's pubic bone (44), wherein the two ends of the fascia (40) are connected to the actuating mechanism (1) and the two positions of the fascia (40) are defined by the two stable settings of the actuating element (8).

## Revendications

1. Mécanisme de commande (1) implantable pouvant être commuté entre deux positions stables pour un implant pouvant être commuté entre deux états ou positions, avec un élément d'appui (2) qui comprend une plaque de montage (3) pour créer un appui sur un os et un élément de commande (8), repoussé par un ressort et mobile par rapport à l'élément d'appui (2), qui s'étend à travers l'élément d'appui (2) et qui est relié, à l'aide d'un câble (51), à un élément d'activation disposé de l'autre côté de l'élément d'appui, déclenchant un changement entre les deux positions de l'implant, **caractérisé en ce que** l'élément d'appui (2) comprend une extrémité coudée (50) dans laquelle le câble (51) est guidée vers l'élément d'activation.

2. Mécanisme de commande selon la revendication 1, **caractérisé en ce que** l'élément d'activation comprend un logement (25) pour les deux extrémités d'un ligament (40) soutenant un conduit corporel.

3. Mécanisme de commande selon la revendication 2, **caractérisé en ce que** le logement (25) comprend une première plaque d'appui (26) et une deuxième plaque d'appui (27) opposées munies de bosses et de creux correspondants.

4. Mécanisme de commande selon la revendication 2, **caractérisé en ce que** le logement (25) comprend une première plaque d'appui (55) centrale et deux plaques d'appui extérieures munies de bosses et de creux appropriés.

5. Mécanisme de commande selon la revendication 3 ou 4, **caractérisé en ce qu'**une partie des bosses se termine par des pointes (28).

6. Mécanisme de commande selon l'une des revendications 3 à 5, **caractérisé en ce qu'**une deuxième partie des bosses et constituée de tiges cylindriques (29) qui s'emboîtent dans des alésages (32).

7. Mécanisme de commande selon la 1, **caractérisé en ce que** le câble (51) est disposé dans une gaine de câble qui comprend des billes (52) disposées les unes à la suite des autres et des disques intermédiaires (53) avec des surfaces d'appui sphériques.

8. Mécanisme de commande selon l'une des revendications 1 à 7, **caractérisé en ce qu'**entre l'élément d'appui (2) et l'élément d'activation, se trouve un soufflet (24).

9. Dispositif implantable pour empêcher l'incontinence urinaire, avec un ligament (40) soutenant le conduit urinaire (43), qui peut être amené, pour soulever la base du conduit urinaire, d'une position inférieure à une position supérieure, **caractérisé en ce que** les deux extrémités du ligament (40) sont reliées à un mécanisme de commande (1), pouvant être fixé au pubis (44), selon l'une des revendications 1 à 8, où les deux positions du ligament (40) sont chacune définies par une position stable de l'élément de commande (8).
